# EUROPEAN PATENT APPLICATION

(11) **EP 2 564 875 A1**
(43) Date of publication of application: **06.03.2013**
(21) Application number: 11775117.2
(22) Date of filing: 28.04.2011
(51) Int. Cl.: A61K 49/04, A61K 9/08, A61K 47/10, A61K 47/26, A61K 47/34, A61K 47/36, A61K 47/38, A61K 47/42

(54) **LIQUID PREPARATION FOR ORAL ADMINISTRATION WHICH CAN BE USED IN CT COLONOGRAPHY, AND COMPOSITION FOR IMAGING OF DIGESTIVE TRACT**

(30) Priority: 30.04.2010 JP 2010105356
(71) Applicant: Ajinomoto Co., Inc., Tokyo 104-8315 (JP)
(72) Inventor: MITSUSHIMA Toru, Kamogawa-shi Chiba 296-0035 (JP)
(74) Representative: Nicholls, Kathryn Margaret
(86) International application number: PCT/JP2011/060384
(87) International publication number: WO 2011/136336

(57) **Abstract**

An liquid preparation for oral administration in digestive tract imaging, wherein: the preparation comprises an iodine compound, an electrolyte, and a water-soluble polymer; and the iodine content is 1.5 to 12.0 mg/ mL.

## Description

### TECHNICAL FIELD

The present invention relates to a liquid preparation for oral administration to be used in the pretreatment of CT colonography, and a composition for digestive tract imaging to be used in the preparation of this liquid preparation for oral administration.
Priority is claimed on Japanese Patent Application No. 2010-105356, filed April 30, 2010, the content of which is incorporated herein by reference.

### BACKGROUND ART

Colon examination by means of computed tomography (CT) (CT colonography) is an examination method which is becoming a common method with the developments of helical CT and multislice CT. In Western countries, this method has already been prevailing as a colon screening test. The CT colonography starts with fecal pat cleansing in the large intestine by means of bowel cleansing, followed by CT image taking in a state where the large intestine lumen is inflated with air, and then the image information is constructed and processed based on a small difference in the X-ray contrast between the colon tissue and the air (for example, refer to Non-Patent Document 1).

CT colonography is capable of re-constructing three dimensional (3D) images such as virtual endoscopy (hereunder, may be referred to as VE) images from digital data of two dimensional (2D) images acquired by image taking, and is capable of displaying these 3D images, by which the large intestine can be diagnosed from various angles. In addition, CT colonography has a potential to take images of the entire abdomen including the large intestine all at once within a short period of time, such as one breath hold only, so that a lesion occurring in another organ differing from the large intestine can also be diagnosed at the same time. Furthermore, CT colonography does not require a highly technical manipulation to adhere barium sulfate to the mucosa of the intestinal tract. For this reason, CT colonography is expected to be conducted more widely in the future because the examination takes a much shorter time and imposes much less burden on the patient, as compared to the enema X-ray examination and the colon endoscopic examination which have been so far conducted.

However, in CT colonography, a fecal pat remaining in the large intestine is more likely to be mistaken as a lesion because the image information is constructed and processed based on a small difference in the X-ray contrast between the digestive tissue and the air, as compared to the colon endoscopic examination in which residues can be visually removed, and the enema X-ray examination in which a contrast between images can be made by using barium sulfate serving as a strong radiographic contrast agent. For this reason, it is necessary to exclude the contents of the intestinal tract in the bowel pretreatment so as to favorably visualize 2D images and 3D images of the large intestine with use of multislice CT, for example, MDCT (Multi Detector-row CT).

As a method for the bowel pretreatment, the intestinal tract cleansing method by means of a non-secretable and non-absorbable oral intestinal tract cleansing liquid is widely used. In the oral intestinal tract cleansing method, an intestinal tract cleansing liquid prepared by dissolving a powdery composition comprising an electrolyte and the like in a predetermined amount, for example, about 2L, of water is taken over 2 to 4 hours. In the bowel pretreatment of CT colonography, an intestinal tract cleansing liquid having been so far used in the colon endoscopic examination and the enema X-ray examination can be adopted. Such an intestinal tract cleansing liquid can be exemplified by: an intestinal tract cleansing liquid which comprises a tonicity agent such as polyethylene glycol and an electrolyte, as main ingredients (for example, refer to Patent Documents 1 and 2); and an intestinal tract cleansing liquid which comprises a combination of a saline laxative such as magnesium citrate, sodium dihydrogen phosphate, disodium hydrogen phosphate, or the like, and a tonicity agent, as an isotonic aqueous solution (for example, refer to Patent Document 3).

However, the problem is that, if such an oral intestinal tract cleansing liquid is used so as to reduce residual feces, the amount of liquid residues is increased. The remaining water and a site pooled with intestinal juice can not be contrasted against the digestive tract tissue, making it impossible to construct examination images thereof. In other words, the remaining water and the site pooled with intestinal juice appear as a totally undetectable region in which any lesion can not be detected at all.

As a method for decreasing such a lesion-undetectable region by reducing liquid residues, for example, there is disclosed a method in which an enterokinesis promoter is used at the time of the oral intestinal tract cleansing (for example, refer to Patent Documents 4 and 5). The amount of liquid residues in the intestinal tract can be decreased by taking the enterokinesis promoter prior to, or at the same time of, taking the oral intestinal tract cleansing liquid.

In addition, attention is drawn to the usefulness of an examination method in which remaining water and pooled liquid in the large intestine are electronically removed by administering a contrast agent together with an intestinal tract cleansing liquid (electronic cleansing) (for example, refer to Patent Documents 6 and 7). The use of the contrast agent makes it possible to visualize the remaining water and the liquid residue pool to thereby examine the lesion-undetectable region.

The concentration of the contrast agent such as an iodine compound in the intestinal tract cleansing liquid is an important factor which governs the ability to make an image of the digestive tract. For example, Patent Document 7 describes that the concentration of the iodine compound content should be set to 15 mg/ mL or higher so as to favorably visualize a liquid residue pool of the intestinal tract cleansing liquid in VE or such 3D images.

However, the iodine compound is so bitter that it is quite difficult to take a large amount of an intestinal tract cleansing liquid containing a high concentration of the iodine compound. Thus, it is usually proposed to take a sufficient amount (for example, 1.5 L or more) of an intestinal tract cleansing liquid so as to sufficiently cleanse the inside of the intestine, and thereafter take a small amount (for example, 350 to 450 mL) of an intestinal tract cleansing liquid which contains the iodine compound so that the iodine content can be adjusted to 15 mg/mL.

### Prior Art Documents

### [Patent Documents]

[Patent Document 1] Japanese Unexamined Patent Application, First Publication No. 2005-187448
[Patent Document 2] Japanese Patent No. 4131266
[Patent Document 3] Japanese Unexamined Patent Application, First Publication No. H05-306221
[Patent Document 4] Japanese Unexamined Patent Application, First Publication No. 2002-53496
[Patent Document 5] PCT International Publication No. WO 2004/004542 pamphlet
[Patent Document 6] Published Japanese Translation No. 2002-539568 of the PCT International Publication
[Patent Document 7] Japanese Unexamined Patent Application, First Publication No. 2005-2006

### [Non-Patent Document]

[Non-Patent Document 1] Liang, J. Z., "3. Virtual colonoscopy: An Alternative Approach to Examination of the Entire Colon", INNERVISION, 2001, Vol. 16, No. 10, pp. 40-44

### [Summary of the Invention]

### [Problems to be Solved by the Invention]

On the other hand, the large intestine has a complicated three dimensional structure. Thus, in the case where a small amount of an intestinal tract cleansing liquid which contains a high concentration of a contrast agent is taken right before completion of taking an intestinal tract cleansing liquid, it may be sometimes difficult for the contrast agent to reach sufficiently deep inside the large intestine. In a state where the majority of the intestinal tract cleansing liquid having been taken from the beginning of starting the intestinal tract cleansing is still pooled in the large intestine, even if a small amount of the intestinal tract cleansing liquid which contains the contrast agent is additionally taken therein, the possibility is low for the contrast agent to evenly reach from the stomach to the small intestine, the large intestine, and even more to the ends of the large intestine. Therefore, the large intestine imaging effect as expected may not be achieved.

The present invention addresses the above-mentioned situations. It is an object of the present invention to provide a liquid preparation for oral administration to be used in the pretreatment of CT colonography for favorably visualizing liquid residues existing deep inside the large intestine, and a composition for digestive tract imaging to be used in the preparation of this liquid preparation for oral administration.

### DISCLOSURE OF INVENTION

The inventors of the present invention have conducted earnest studies so as to solve the above-mentioned problems. As a result, they have discovered that it is possible to carry out the examination with effective visualization of the liquid residues which partially exist in the large intestine by: administering an intestinal tract cleansing liquid which contains a low content concentration of a contrast agent continuously from the beginning of the administration, then taking a CT image, and reading the thus taken 2D image and the like; instead of the conventional two-step administration method of administering an intestinal tract cleansing liquid which does not contain a contrast agent and thereafter administering an intestinal tract cleansing liquid which contains a high concentration of a contrast agent. This has led to the completion of the present invention.

That is, a first aspect of the present invention is a liquid preparation for oral administration in digestive tract imaging, wherein: the preparation comprises an iodine compound, an electrolyte, and a water-soluble polymer; and the iodine content is 1.5 to 12.0 mg/ mL.
A second aspect of the present invention is a liquid preparation for oral administration according to the first aspect, wherein the preparation further comprises one or more types of ingredients selected from the group consisting of a sweetening agent and a perfume.
A third aspect of the present invention is a liquid preparation for oral administration according to either one of the first and second aspects, wherein the water-soluble polymer is one or more types of water-soluble polymers selected from the group consisting of polyethylene glycol, polydextrose, dextran, dextrin, hydroxyethyl starch, gum arabic, pullulan, pectin, albumin, and carboxymethyl cellulose.
A fourth aspect of the present invention is a liquid preparation for oral administration according to any one of the first to third aspects, wherein the mixed amount of the electrolyte is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L.
A fifth aspect of the present invention is a liquid preparation for oral administration according to any one of the first to fourth aspects, wherein the perfume is a citrus-based perfume.
A sixth aspect of the present invention is a liquid preparation for oral administration according to any one of the first to fifth aspects, wherein the sweetening agent comprises one or more types of saccharides selected from the group consisting of sucrose, sorbitol, xylitol, erythritol, mannitol, trehalose, lactitol, lactulose, maltitol, palatinose, raffinose, and glycerin.
A seventh aspect of the present invention is a liquid preparation for oral administration according to any one of the first to sixth aspects, wherein: the preparation is to be taken from the beginning of intestinal tract cleansing as a pretreatment of CT colonography; a CT image is taken after the completion of the intestinal tract cleansing; and digestive tract tissue of the large intestine is visualized from the thus taken CT 2D image.
An eighth aspect of the present invention is a composition for digestive tract imaging for use in intestinal tract cleansing, wherein: the composition comprises an iodine compound, an electrolyte, and a water-soluble polymer; and the iodine content is 1.5 to 12.0 mg/ mL when dissolved in water.
A ninth aspect of the present invention is a composition for digestive tract imaging according to the eighth aspect, wherein: the content of the electrolyte when dissolved in water is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L; and the composition comprises one or more types of water-soluble polymers selected from the group consisting of polyethylene glycol, polydextrose, dextran, dextrin, hydroxyethyl starch, gum arabic, pullulan, pectin, albumin, and carboxymethyl cellulose, as the water-soluble polymer; or, one or more types of saccharides selected from the group consisting of sucrose, sorbitol, xylitol, erythritol, mannitol, trehalose, lactitol, lactulose, maltitol, palatinose, raffinose, and glycerin, as saccharide(s).
A tenth aspect of the present invention is a composition for digestive tract imaging according to the eighth aspect, wherein; the content of the electrolyte when dissolved in water is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L; and the composition comprises one or more types of water-soluble polymers selected from the group consisting of polyethylene glycol, polydextrose, dextran, dextrin, hydroxyethyl starch, gum arabic, pullulan, pectin, albumin, and carboxymethyl cellulose, as the water-soluble polymer; and one or more types of saccharides selected from the group consisting of sucrose, sorbitol, xylitol, erythritol, mannitol, trehalose, lactitol, lactulose, maltitol, palatinose, raffinose, and glycerin, as saccharide(s).
An eleventh aspect of the present invention is a composition for digestive tract imaging according to the eighth aspect, wherein the composition further comprises one or more types of ingredients selected from the group consisting of a sweetening agent and a perfume.
A twelfth aspect of the present invention is a composition for digestive tract imaging according to any one of the eighth to eleventh aspects, wherein: the intestinal tract cleansing is a pretreatment of CT colonography; and 0.5 to 4L of the water solution is orally administered from the beginning of the intestinal tract cleansing; a CT image is taken after the completion of the intestinal tract cleansing; and digestive tract tissue of the large intestine is visualized from the thus taken CT 2D image.
A thirteenth aspect of the present invention is a pretreatment agent for CT colonography examination, wherein: the agent comprises a combination of an oral intestinal tract cleansing agent and an iodine contrast agent; and the iodine content is adjusted to 1.5 to 12.0 mg/ mL and thereafter the intestinal tract cleansing is begun by starting oral administration.
A fourteenth aspect of the present invention is a use of a liquid preparation for oral administration in digestive tract imaging, wherein: the preparation comprises an iodine compound, an electrolyte, and a water-soluble polymer; and the iodine content is 1.5 to 12.0 mg/ mL.
A fifteenth aspect of the present invention is a use of a liquid preparation for oral administration in digestive tract imaging, wherein: the preparation comprises an iodine compound, an electrolyte, a water-soluble polymer, and one or more types of ingredients selected from the group consisting of a sweetening agent, and a perfume; and the iodine content is 1.5 to 12.0 mg/ mL.
A sixteenth aspect of the present invention is a use of a liquid preparation for oral administration in intestinal tract cleansing, wherein: the preparation comprises an iodine compound, an electrolyte, and a water-soluble polymer; and the iodine content is 1.5 to 12.0 mg/ mL.
A seventeenth aspect of the present invention is a use of a liquid preparation for oral administration in intestinal tract cleansing, wherein: the preparation comprises an iodine compound, an electrolyte, a water-soluble polymer, and one or more types of ingredients selected from the group consisting of a sweetening agent, and a perfume; and the iodine content is 1.5 to 12.0 mg/ mL.
An eighteenth aspect of the present invention is an oral use of a combination of an oral intestinal tract cleansing agent and an iodine contrast agent in a pretreatment for CT colonography examination, wherein: the use of the combination is for intestinal tract cleansing and digestive tract imaging; and the content of the iodine compound is 1.5 to 12.0 mg/ mL in the combination of the oral intestinal tract cleansing agent and the iodine contrast agent.
A nineteenth aspect of the present invention is a kit for digestive tract imaging or intestinal tract cleansing, wherein: the kit comprises an iodine compound, an electrolyte, and a water-soluble polymer; and the iodine content is 1.5 to 12.0 mg/ mL.
A twelfth aspect of the present invention is a kit for digestive tract imaging or intestinal tract cleansing, wherein: the kit comprises an iodine compound, an electrolyte, a water-soluble polymer, and one or more types of ingredients selected from the group consisting of a sweetening agent and a perfume; and the iodine content is 1.5 to 12.0 mg/ mL.
A twenty first aspect of the present invention is a method for imaging a digestive tract of a target, wherein the method comprises orally administering the target with a liquid preparation comprising an iodine-containing compound, an electrolyte, and a water-soluble polymer, with an iodine content of 1.5 to 12.0 mg/ mL, at an effective amount for the digestive tract imaging.
A twenty second aspect of the present invention is a method for imaging a digestive tract of a target, wherein the method comprises orally administering the target with a liquid preparation comprising an iodine-containing compound, an electrolyte, a water-soluble polymer, and one or more types of ingredients selected from the group consisting of a sweetening agent and a perfume, with an iodine content of 1.5 to 12.0 mg/ mL, at an effective amount for the digestive tract imaging.

### [Effects of the Invention]

By using the liquid preparation for oral administration of the present invention in the pretreatment of CT colonography, a lesion-undetectable region derived from remaining water of the intestinal tract cleansing liquid or a liquid residue pool of intestinal juice that often appears in the large intestine lumen after the intestinal tract cleansing can be rendered an image-readable region.
In addition, by using the composition for digestive tract imaging of the present invention, the liquid preparation for oral administration of the present invention can be prepared as a handy and easily-drinkable preparation.

### BRIEF DESCRIPTION OF THE DRAWINGS

FIG. 1 shows CT imaging results of plastic bottles injected with colon cleansing solution mixes in Example 1.
FIG. 2 shows simple roentgenographic results of the 1% colon cleansing solution mixes injected into plastic bottles taken after 48 hours in Example 1.
FIG. 3 shows endoscopic images of an examinee with no residual feces and only transparent drained water in Example 1.
FIG. 4 shows endoscopic images of an examinee with a little residual feces in Example 1.
FIG. 5 shows scanographic images just before CT image taking after insufflation of CO₂ in Example 1.
FIG. 6 shows 2D images obtained as a result of CT image taking in Example 1.
FIG. 7 shows 2D images obtained as a result of CT image taking in Example 1.

### BEST MODE FOR CARRYING OUT THE INVENTION

The liquid preparation for oral administration of the present invention is an iodine compound-containing liquid preparation for oral administration in digestive tract imaging, wherein: the iodine content is 1.5 to 12.0 mg/ mL; and the preparation comprises an electrolyte, and a water-soluble polymer, and further comprises one or more types of ingredients selected from the group consisting of a sweetening agent and a perfume.

The iodine compound to be contained in the liquid preparation for oral administration of the present invention is not specifically limited as long as it is a water-soluble organic compound which contains one or more iodine atoms in a molecule and can be used as a contrast agent. Such a water-soluble organic iodine compound can be exemplified by a triiodide benzene in which iodine atoms are bonded to the second, fourth, and sixth positions of a benzene ring. The iodine compound to be contained in the liquid preparation for oral administration of the present invention may be either an ionic compound or an anionic compound.

Regarding specific examples of such compounds, amidotrizoic acid (chemical name: 3,5-diacetamino-2,4,6-triiodobenzoic acid), meglumine sodium amidotrizoate, meglumine amidotrizoate, sodium iotalamate, meglumine iotalamate, meglumine iotroxate, iotrolan, ioxaglic acid, ioxilan, iopamidol, iopromide, iohexol, ioversol, iomeprol, and the like, can be enumerated.

The iodine compound can be selected by appropriately considering the background of the patient, such as an allergic constitution and like, because the respective compounds may cause a side effect such as an anaphylactoid reaction.

In particular, it is preferable to use meglumine sodium amidotrizoate for a patient with a little risk of the occurrence of a side effect, such as an allergic constitution and the like, in terms of the pharmaceutical stability, the cost, and the like,.

The iodine compound should be contained in the liquid preparation for oral administration of the present invention so that the iodine content is 1.5 to 12.0 mg/mL. As described above, in the conventional two-step administration method, an iodine compound-containing intestinal tract cleansing liquid having an iodine content of 15 mg/ mL or higher is administered. However, the iodine compound is so bitter that it is quite difficult to take a solution having such a high iodine content after already taking a large amount of a cleansing liquid. The liquid preparation for oral administration of the present invention is prepared so that the iodine content has a relatively low concentration of 12.0 mg/ mL or lower, and preferably from 3.0 to 7.0 mg/ mL. Thus, it is possible to administer the liquid preparation for oral administration of the present invention continuously from the beginning of the pretreatment without imposing a burden on the patient. For this reason, it is possible to easily administer the liquid preparation for oral administration of the present invention at a sufficient amount for cleansing the intestinal tract as a pretreatment of CT colonography, similarly to an intestinal tract cleansing liquid containing no iodine compound.

In the liquid preparation for oral administration of the present invention, it is possible to hold a sufficient amount of the iodine compound in the whole interior of the intestinal tract, particularly, in a liquid residue pool existing in the intestinal tract deep inside the large intestine, as well as adequately cleansing the intestinal tract, by conducting the pretreatment with use of the liquid preparation for oral administration of the present invention instead of an intestinal tract cleansing liquid containing no iodine compound. In other words, in a CT 2D image having been captured after conducting the pretreatment with use of the liquid preparation for oral administration of the present invention, the image of a liquid residue pool reached by this liquid preparation for oral administration, particularly, a liquid residue pool in the intestinal tract deep inside the large intestine, can be efficiently visualized in a detectable manner.

As described in Patent Documents 6 and 7, the electronic cleansing needs to be done so that the 2D images captured by CT image taking can be precisely reconstructed into 3D images. Moreover, in order to efficiently conduct the electronic cleansing, the brightness of the liquid residue pool has to sufficiently high. For this reason, in Patent Document 7, in order to visualize the liquid residue pool, the iodine content is set to 15 mg/ mL or higher in the intestinal tract cleansing liquid to be administered to the examinee upon the pretreatment of CT colonography.

The iodine content of the liquid preparation for oral administration of the present invention is 12.0 mg/ mL or lower, which is disadvantageous for the liquid residue pool in the CT-captured image to have a required brightness to conduct efficient electronic cleansing. However, it is possible in the CT 2D image itself to sufficiently visualize a lesion in the liquid residue pool, and to examine the whole intestinal tract by reading this image.
By so doing, it becomes possible with CT colonography to precisely examine a lesion in a shadow of a curvature, a semilunar fold, or a Bauhin valve of the intestinal tract, which has been so far difficult to examine in the endoscopic examination, although the endoscopic examination has been employed as a detailed examination in the conventional colon cancer screening test.

The water-soluble polymer to be contained in the liquid preparation for oral administration of the present invention can be exemplified by polyethylene glycol, polydextrose, dextran, dextrin, hydroxyethyl starch, gum arabic, pullulan, pectin, carboxymethyl cellulose, and the like. Of these, one or more types of polymers selected from the group consisting of polyethylene glycol, polydextrose, dextran, hydroxyethyl starch, gum arabic, pullulan, and pectin is/are preferred in terms of the pharmaceutical stability and the like.

It is particularly preferable that the liquid preparation for oral administration of the present invention contains polyethylene glycol as a water-soluble polymer. The molecular weight of the above-mentioned polyethylene glycol is preferably from 2000 to 8000. It is more preferable to adopt polyethylene glycol within a range from 3000 to 7000.

The term "electrolyte" refers to a substance that becomes ionic by dissociation in a solution, examples of which can be given by Na⁺, K⁺, Ca⁺⁺, Mg⁺⁺, Cl⁻, HCO₃⁻, SO₄⁻⁻,HPO₄⁻⁻, an organic acid group, an organic base group, and the like. The electrolyte to be contained in the liquid preparation for oral administration of the present invention can be exemplified by the same electrolytes for use in an electrolyte infusion solution for intravenous administration, and the like. More specifically, the electrolyte can be exemplified by, respectively: sodium chloride, sodium acetate, sodium citrate, sodium dihydrogen phosphate, disodium hydrogen phosphate, sodium sulfate, sodium lactate, or the like, as a sodium ion source; potassium chloride, potassium acetate, potassium citrate, potassium dihydrogen phosphate, dibasic potassium phosphate, potassium sulfate, potassium, lactate, or the like, as a potassium ion source; calcium chloride, calcium gluconate, calcium pantothenate, calcium lactate, calcium acetate, calcium glycerophosphate, or the like, as a calcium ion source; magnesium sulfate, magnesium chloride, magnesium acetate, magnesium citrate, or the like, as a magnesium ion source; sodium dihydrogen phosphate, disodium hydrogen phosphate, sodium glycerophosphate, or the like, as a phosphate ion source; sodium chloride, potassium chloride, calcium chloride, magnesium chloride, or the like, as a chlorine ion source; and sodium hydrogen carbonate or the like as a bicarbonate ion source. These compounds may be in hydrated forms.

The osmotic pressure of the liquid preparation for oral administration of the present invention is preferably from 200 to 440 mOsm/L. By setting the osmotic pressure within this range, it is possible to minimize fluctuations of the serum electrolyte balance and the osmotic pressure in the body caused by the administration of this liquid preparation for oral administration. It is more preferable to adjust the osmotic pressure of the liquid preparation for oral administration of the present invention, to a range of isotonic, or nearly isotonic, to 280 to 320 mOsm/L.

The liquid preparation for oral administration of the present invention may also be a preparation to be taken with water or the like so that the osmotic pressure in the intestinal tract is adjusted, similarly to the intestinal tract cleansing liquid described in, for example, Patent Document 2. If the liquid preparation for oral administration of the present invention is taken with water or the like in this way, it is also possible to adjust, for example, the osmotic pressure of the liquid preparation for oral administration of the present invention to be higher than the above-mentioned range of the osmotic pressure, for example, at 700 mOsm/L or lower.

In the liquid preparation for oral administration of the present invention, mainly the electrolyte and the water-soluble polymer play a function as an osmotic pressure adjuster. Accordingly, these ingredients and their mixed amounts should be appropriately selected so that the osmotic pressure of the liquid preparation for oral administration is set within the above-mentioned range.

In addition to the electrolyte and the water-soluble polymer, a saccharide may also be added to the liquid preparation for oral administration of the present invention. Regarding a particularly preferable liquid preparation for oral administration for the pretreatment of CT colonography to be conducted as a screening test, it is preferable to add a mixture of an electrolyte and a saccharide for the reason of better acceptability for the patient, including the pharmaceutical stability, the easiness to drink, and the like.

The saccharide to be contained in the liquid preparation for oral administration of the present invention may be either sugar or sugar alcohol. The saccharide to be contained in the liquid preparation for oral administration of the present invention can be specifically exemplified by sucrose, sorbitol, xylitol, erythritol, mannitol, trehalose, lactitol, lactulose, maltitol, palatinose, raffinose, glycerin, and the like. Of these, one or more types of saccharides selected from the group consisting of xylitol, sorbitol, lactulose, lactitol, and raffinose is/are particularly preferred for the reason of the acceptability for the patient, including the pharmaceutical stability, the easiness of administration, and the like.

The sum of the mixed amounts of the water-soluble polymer and the electrolyte in the liquid preparation for oral administration of the present invention, or the total sum of the mixed amounts of the water-soluble polymer, the electrolyte, and the saccharide, is, for example, from 2 to 40 gram, and preferably from 10 to 30 gram in 400 mL of the liquid preparation. In order to keep the serum electrolyte balance by offsetting the amount of the electrolyte to be absorbed from the intestinal tract and the amount of the electrolyte to be secreted into the intestinal tract, it is preferable to add an electrolyte so that Na+ accounts for 30 to 150 mEq/L, K+ accounts for 3 to 20 mEq/L, Cl- accounts for 20 to 70 mEq/L, and HCO3- accounts for 10 to 50 mEq/L in the liquid preparation for oral administration of the present invention. In order to suppress the absorption of water and electrolyte into the intestinal tract, it is preferable to add a magnesium ion, a sulfate ion, or such a poorly absorbable ion, in addition to the above-mentioned electrolytes, to the liquid preparation for oral administration. In the case of a magnesium ion, it is preferable to mix it by adjusting so that the magnesium ion concentration in the liquid preparation for oral administration is from 40 to 120 mEq/L. In the case of a sulfate ion, it is preferable to mix it by adjusting so that the sulfate ion concentration in the liquid preparation for oral administration is from 40 to 120 mEq/L. If a saccharide is used in addition to the electrolyte and the water-soluble polymer, the dose of the saccharide is from 2 to 40 gram, and preferably from 5 to 10 gram, in 400 mL of the liquid preparation for oral administration.

The iodine compound is quite bitter. Moreover, the water-soluble polymer and the electrolyte often have a peculiar taste and odor. Therefore, it is preferable to apply taste-masking and odor-masking means to the liquid preparation for oral administration of the present invention. Specifically, a sweetening agent and a perfume can be mixed.

The perfume to be contained in the liquid preparation for oral administration of the present invention is not specifically limited as long as it is a perfume that can mask an odor coming from the water-soluble polymer and the electrolyte when the liquid preparation for oral administration is taken orally. In the present invention, a perfume for food is appropriate and a fruit perfume is particularly appropriate. Such a fruit perfume can be exemplified by perfumes of lemon, orange, grapefruit, lemon lime, mandarin orange, grape, strawberry, cherry, apple, apricot, raspberry, and the like.
Of these, citrus-based liquid perfumes of such as lemon, orange, grapefruit, and lemon lime, are most appropriate as they offer refreshing tastes. As for the liquid perfume, an essential oil produced by compression or steam distillation from these fruits can be used. In addition, as for the citrus-based perfume, limonene, citral, citronellal, linalool, octanal, and the like, can be produced solely or as a mixture. Of these, it is preferable to adopt a citrus-based perfume containing limonene. Such perfumes are commercially available from perfume manufacturers, and it is practical to use them. The term "liquid perfume" includes water-soluble liquid perfumes produced by extracting or dissolving the perfume component with hydroalcohol or the like, and oil-soluble liquid perfumes produced by dissolving the perfume component with an oil-based solvent. Furthermore, the above-mentioned term "liquid perfume" also includes liquid perfumes having a preservative or the like added to the perfume component (an essential oil). Regarding the liquid perfume, it is preferable to adopt an oil-soluble or essential oil-derived perfume, rather than a water-soluble liquid perfume, in terms of the preservation stability.

If a perfume is used, it is necessary to adjust the dose with consideration of the volatilization in each production process, the adsorption into the package, the transmission dissipation from the package, and the like. The preferred dose is from 0.001 to 0.3 % by mass of the liquid preparation for oral administration. In the present invention, the perfume is directly adsorbed into the electrolyte powder.
It should be avoided to previously adsorb and mix the perfume into the saccharide because there is a risk of generating hydrogen gas and methane gas.

The sweetening agent to be used in the present invention is a sweetening agent to make it easy to drink a large amount of the liquid preparation for oral administration. The sweetening agent should be a sweetening agent which does not generate, or generates quite small amounts of, hydrogen gas and methane gas in the intestine. However, it is possible to vigorously add a saccharide, specifically, a sucrose (refined white soft sugar), so as to improve the easiness to drink the liquid preparation and to improve the energy supply, unless an endoscopic examination is to be performed after taking the CT colonographic images, or an operation to electrically excise a polyp is applied during the endoscopic examination (endoscopic polypectomy). As the sweetening agent which does not generate, or generates quite small amounts of, hydrogen gas and methane gas in the intestine, specifically speaking, it is preferable to use saccharine, saccharine sodium, acesulfame-K, cyclamate (sodium cyclohexylsulfamate), aspartame, and the like, either solely or as a combination. The preferred dose of these sweetening agents is from 0.001 to 3 % by mass, and more preferably from 0.01 to 0.3 % by mass, of the liquid preparation for oral administration.

Other ingredients can also be contained in the liquid preparation for oral administration of the present invention, as long as the effect of the present invention is not impaired. For example, one or more types of ingredients selected from the group consisting of ascorbic acid and salts thereof (hereunder, may be referred to ascorbate ingredient) may be contained in the liquid preparation for oral administration of the present invention. For example, it is preferable to add the ascorbate ingredient within a range of 4 to 15 g per liter of the liquid preparation for oral administration of the present invention.

Preferred ascorbic acid salts are alkali metal salts and alkaline-earth metal salts, which can be exemplified by sodium ascorbate, potassium ascorbate, magnesium ascorbate, and calcium ascorbate. Particularly preferred ascorbic acid salt is sodium ascorbate. As the ascorbate ingredient, it is preferable to contain both the ascorbic acid and one or more types of salt(s) thereof. It is more preferable that the ascorbic acid and the salt(s) thereof are present within a range from 1:9 to 9:1 in terms of the weight ratio. The ascorbic acid and the salt(s) thereof could be provided in hydrated forms in practice. If such hydrated forms are adopted, the terms "weight" and "weight ratio" referred to herein respectively mean the weight and the weight ratio of the ascorbic acid and the salt(s) thereof excluding the weight and the weight ratio of the hydration water. If these hydrated forms are adopted, the range of the weight ratio of the ascorbic acid and the salt(s) thereof is preferably from 2:8 to 8:2, more preferably from 3:7 to 7:3, and yet more preferably from 4:6 to 6:4. For example, they can exist at a weight ratio of 4.7:5.9.

As a pretreatment of CT colonography, the intestinal tract can be cleansed by administering the liquid preparation for oral administration of the present invention. Specifically speaking, in the pretreatment method to be conducted prior to the CT image taking, 0.5 to 4L, and preferably 1 to 2L of the liquid preparation for oral administration of the present invention is orally administered before taking the CT image. Thereafter, the CT image is taken by a usual method, and the digestive tract tissue of the large intestine is visualized from the thus taken CT 2D image. By so doing, the CT colonography can be carried out. By administering the liquid preparation comprising an iodine compound from the beginning of the intestinal tract cleansing, the iodine compound can be held in all the liquid residues deep inside the large intestine, and a lesion-undetectable region derived from a liquid residue pool or the like can be reduced.

It is preferable to administer the liquid preparation for oral administration of the present invention at a rate of about 1L per hour.
By administering it at such a rate, the contents of the intestinal tract are excreted. If a patient suffers from constipation with less than four times of defecation per week on average, the contents of the intestinal tract can be excreted by administering 200 to 300 mL of an intestinal tract cleansing liquid not containing an iodine compound continuously for three to five days for the purpose of improving the passage of stool, followed by oral administration of 1 to 2L of the liquid preparation for oral administration of the present invention after improving the passage of stool.

The liquid preparation for oral administration of the present invention may be a liquid preparation for oral administration produced by adding an iodine contrast agent having an iodine compound as an active ingredient, to a liquid type of oral intestinal tract cleansing agent (intestinal tract cleansing liquid) for use in intestinal tract cleansing as a pretreatment of the colon endoscopic examination, the enema X-ray examination, and the CT colonography. That is, an intestinal tract cleansing liquid produced by combining an iodine contrast agent with an oral intestinal tract cleansing agent and adjusting the iodine content to 1.5 to 12.0 mg/ mL, is used as a pretreatment agent for the CT colonography examination. By starting the oral administration of this pretreatment agent, the intestinal tract cleansing can be commenced.

Such an intestinal tract cleansing liquid is not specifically limited as long as the intestinal tract cleansing liquid contains an electrolyte and a water-soluble polymer, although it is preferable that the intestinal tract cleansing liquid contains one or more types of ingredients selected from the group consisting of a sweetening agent and a perfume, and has an osmotic pressure of 200 to 440 mOsm/L. Specific examples thereof include: an intestinal tract cleansing liquid which comprises a composition having a combination of polyethylene glycol and an electrolyte (for example, "Niflec (registered trademark)" (a product of Ajinomoto Pharma) related to the invention disclosed in Japanese Unexamined Patent Application, First Publication No. H01-125319); an intestinal tract cleansing liquid which comprises a composition having a combination of at least one type of water-soluble polymer selected from dextran, dextrin, hydroxyethyl starch, polydextrose, gum arabic, and pectin, and an electrolyte (Japanese Unexamined Patent Application, First Publication No. H02-25424, and Japanese Unexamined Patent Application, First Publication No. H03-206046); an intestinal tract cleansing liquid which comprises a composition having a combination of either erythritol or xylitol, and an electrolyte (Japanese Unexamined Patent Application, First Publication No. H03-284620); an intestinal tract cleansing liquid which comprises a composition having a combination of fructo-oligosaccharide and an electrolyte (Japanese Unexamined Patent Application, First Publication No. H03-291228); a composition which comprises at least one type of saccharide selected from lactitol, maltitol, and carboxymethyl cellulose, and an electrolyte (Japanese Unexamined Patent Application, First Publication No. H05-255092); an intestinal tract cleansing liquid in a form of an isotonic aqueous solution, which is produced by using magnesium citrate (Japanese Unexamined Patent Application, First Publication No. H05-306221), sodium dihydrogen phosphate, disodium hydrogen phosphate, or such a saline laxative, and a tonicity agent; and the like.

The liquid preparation for oral administration of the present invention can be prepared by such that: a concentrated liquid containing respective ingredients is prepared in advance, and then diluted with an appropriate amount of water at the time of administration. The concentrated liquid of the respective ingredients may be in a packed form filled in an aluminum can, a flexible container, or a rigid or semi-rigid container. In this case, it is needless to say that the diluent should be disinfected or sterilized by a usual method (Japanese Unexamined Patent Application, First Publication No. H09-58747, Japanese Unexamined Patent Application, First Publication No. H08-253220, and the like).

In addition, the preparation can also be made by such that: respectively powderized, tableted, or granulated ingredients are each prepared, and these are dissolved with an appropriate amount of water at the time of administration. It is also possible such that: a composition having a mixture of two or more types of ingredients is prepared in advance, and then this composition and other ingredients are dissolved with an appropriate amount of water at the time of administration.

For example, the liquid preparation for oral administration of the present invention can be prepared by dissolving a powder preparation which comprises an electrolyte, a water-soluble polymer, a sweetening agent, and a liquid perfume, and which is capable of being dissolved with a predetermined amount of water to thereby prepare an intestinal tract cleansing liquid whose osmotic pressure is from 200 to 440 mOsm/L (for example, the above-mentioned "Niflec (registered trademark)" related to the invention disclosed in the Japanese Unexamined Patent Application, First Publication No. H01 -125319), and a contrast agent having an iodine compound as an active ingredient, with a predetermined amount of water altogether at the time of administration. It is also possible to use an intestinal tract cleansing agent having been prepared in a form of a tablet or granules, instead of the above-mentioned powder preparation.

Besides, it is also possible such that: a composition comprising an iodine compound, an electrolyte, and a water-soluble polymer (and a saccharide if necessary) is prepared in advance, and then this composition and other ingredients such as a sweetening agent and a perfume are dissolved with an appropriate amount of water at the time of administration. A liquid preparation for oral administration having a desired taste and flavor can be more easily prepared by such that: active ingredients such as an intestinal tract cleansing agent and a contrast agent are prepared in advance as one composition, and a sweetening agent, a perfume, and such ingredients having a taste-masking or an odor-masking function are individually set so as to be adjustable.

A composition which contains at least an iodine compound, an electrolyte, and a water-soluble polymer, out of the ingredients constituting the liquid preparation for oral administration of the present invention, is referred to as the composition for digestive tract imaging of the present invention. In other words, the composition for digestive tract imaging of the present invention contains an iodine compound, an electrolyte, and a water-soluble polymer, and is capable of preparing an aqueous solution having an iodine content of 1.5 to 12.0 mg/ mL, preferably 1.5 to 7.0 mg/ mL, and more preferably 3.0 to 5.0 mg/ mL, by dissolution or dilution with water. A composition which also contains a saccharide, a sweetening agent, a perfume, and the like, is also categorized as the composition for digestive tract imaging of the present invention. In addition, the osmotic pressure of the aqueous solution produced by dissolving or diluting the composition for digestive tract imaging of the present invention with water is preferably from 200 to 440 mOsm/L.

The composition for digestive tract imaging of the present invention is preferably a composition for digestive tract imaging prepared by mixing ingredients but for the iodine contrast agent constituting the liquid preparation for oral administration of the present invention, after adjusting their particle diameters so that the respective ingredients can be mixed.

Such a composition containing all the ingredients is preferably filled and hermetically sealed in an airtight container before placing on the market. In particular, in order to keep the easiness of administration and the convenience of use, it is preferable to adjust the amounts of the contents in one package so that all the ingredients constituting the liquid preparation for oral administration of the present invention, such as the iodine compound, can be all mixed or dissolved in 1 L or 2L when used.

The composition for digestive tract imaging of the present invention is filled in a package such as a bag or a can formed of aluminum or such a metal with a lining of a thermoplastic resin, or a plastic package formed of a thermoplastic resin. In this case, a limonene-containing perfume is apt to be adsorbed particularly in a polyolefin-based thermoplastic resin, or to be dissipated by transmission through the polyolefin-based thermoplastic resin. Thus, the package has to be prepared so that a sufficient amount of the perfume can remain at the time of use. The amount of adsorption and the amount of transmission dissipation of the perfume are dependent on the thickness of the resin layer as well as on the area of the inner surface of the package. Thus, the thickness of the resin layer or the like has to be set within a range where the sealing strength of the package would not be lowered and a range where a sufficient amount of the perfume can remain at the time of use. It is particularly preferable to make the package so that the amount of adsorption or the amount of transmission dissipation of limonene per one package will not be greater than 1000 mg by the time of use.

The composition for digestive tract imaging of the present invention can be administered by transferring from the package to a container and dissolving with water. However, it would be more convenient if the package itself is a plastic container which can accommodate a required dissolving water (for example, 2L), and the composition is administered by directly pouring the dissolving water into the package and dissolving at the time of use, because there is no need for preparing a separate container for use in the dissolution. The plastic container in this case is preferably formed of a polyolefin-based thermoplastic resin. The polyolefin-based thermoplastic resin can be exemplified by polyethylene and polypropylene. Flexible polyolefin has excellent adhesiveness between resins necessary for molding the container, while it has high adsorptivity/transmissivity of a limonene-containing perfume gas. On the other hand, rigid polyolefin has low adsorptivity/transmissivity of the gas, while it has unfavorable adhesiveness between resins. Accordingly, in order to satisfy both the requirements for the molding and for the adsorptivity/transmissivity of the perfume gas, it is possible to make a package which has low adsorptivity/transmissivity of the perfume gas and excellent adhesiveness (molding property) as a whole, and, what is more, which is a large volume package that can act as a dissolving container, if a flexible polyolefin is used for the inner layer (the surface to be contacted with the composition for digestive tract imaging) and a rigid polyolefin is superposed thereon. Furthermore, particularly preferred is a container having three layers in total, in which a flexible polyolefin is used for the inner most layer (the surface to be contacted with the composition for digestive tract imaging), a rigid polyolefin is superposed on the exterior thereof, and a flexible polyolefin is superposed on the further exterior thereof.

It is preferable to use a flexible polyethylene, particularly a linear polyethylene, for the inner most layer. Regarding the thickness of the inner most layer in this case, it is preferable to form the layer from a polyolefin-based thermoplastic resin having a thickness of a 100 µm or thinner, and preferably 50 µm, so as to suppress the reduction of the limonene-containing perfume caused by adsorption or transmission dissipation. In the case where the plastic package is formed of two layers consisting of an inner layer made of a flexible polyolefin and an outer layer made of a rigid polyolefin, it is preferable that the total thickness is from 70 to 200 µm, and the thicknesses of the inner layer and the outer layer are respectively from 35 to 100 µm. In the case where the plastic package is formed of three layers consisting of a flexible polyolefin as the inner most layer, a rigid polyolefin superposed on the exterior thereof, and a flexible polyolefin superposed on the further exterior thereof, it is preferable that the total thickness is from 70 to 200 µm, and the thicknesses of the respective layers are from 15 to 70 µm, and particularly from 20 to 40 µm.

In order to achieve the both purposes of preventing the lowering of the content of sodium hydrogen carbonate due to the over-time generation of carbon dioxide gas, as well as preventing the dissipation of limonene from the package, among the active ingredients, the degree of gas transmission through the package is preferably not higher than 20 cc/m²·day·atm (25°C).

It is preferable to form a package the strength of which has been improved so as to endure drop impact or prodding impact against the package, by additionally superposing a thermoplastic resin such as polyethylene terephthalate or polyamide on the further exterior of the above-mentioned double- or triple- layered polyethylene laminate.

The shape of the package is not specifically limited. However, if the package is also used as a plastic container which can accommodate the dissolving water, it is preferable that the package has an inlet for the dissolving water and a bottom face so that the package can stand during the dissolution with the dissolving water. With consideration of the convenience of production, preferred is a package having two approximately triangular and oblong lateral walls whose height is longer than the side length of the bottom face, an inlet attached to the top of the lateral walls, and the bottom face shaped like a camellia leaf attached to the bottom of the lateral walls. These two lateral walls and the bottom face are made of a bendable plastic and the inlet is made of a rigid plastic. The inlet is provided with a cap. By additionally having a structure such that these two lateral walls and the bottom face are made of a bendable plastic so that the bottom face can be bent inward until the dissolving water is poured, the entire structure can be kept compact by excluding air from the package (container) until the dissolving water is poured. Thus, such a structure is convenient for storage and transportation. It is preferable that these two lateral walls are made of a transparent or semi-transparent plastic so that the amount of the content can be checked by eye. By putting scale marks to show the amount of the dissolving water on these lateral walls, there is no need for a scale when pouring the dissolving water, and the dose can be checked by eye upon administration.

The composition for digestive tract imaging of the present invention can be prepared by mixing predetermined ingredients in a conventionally known method. If the composition for digestive tract imaging of the present invention is filled in a package, it is preferable to exclude air as much as possible, because active ingredients that are unstable in oxygen can be kept from quality deterioration over time, and the package can be kept non-bulky, which makes it convenient for storage and transportation. In addition, if long-term storage is needed, this package can be additionally set and hermetically sealed in a non-gas-transmissive pouch formed of an aluminum laminate film or the like.

It is preferable that the respective ingredients, the composition, and the like, for use in the preparation of the liquid preparation for oral administration of the present invention, such as the composition for digestive tract imaging of the present invention, are prepared as one kit or a packaged product. The production of such a kit or a packaged product is not specifically limited, and can be appropriately carried out by a usual method.
In other words, it suffices if at least the respective ingredients and the composition for use in the preparation of the liquid preparation for oral administration of the present invention are contained in the form of one package. It is preferable to put an instruction which explains the order and how to take the preparation, the symptoms for which administration should be stopped, and a note of caution which explains how to deal with side effects if these happen, in the kit and packaged product. It is also possible to additionally put a cup, a check sheet of dosage, a timer, a dissolving liquid, and the like, in the kit and packaged product.

As an example, a powder preparation which contains an electrolyte, a water-soluble polymer, a sweetening agent, and a liquid perfume, and which is capable of being dissolved with a predetermined amount of water to thereby prepare an intestinal tract cleansing liquid whose osmotic pressure is from 200 to 440 mOsm/L, is filled in a plastic container (refer to Japanese Unexamined Patent Application, First Publication No. H04-259461). This container is detachably attached with a blister pack which separately accommodates a liquid preparation or a tablet containing an iodine compound, instructions which explain the procedure of administration, and a note of caution which explains the symptoms of side effects, and how to deal with them. In addition, a useful form is that the above-mentioned plastic container attached with the blister pack is inserted in, or attached to the outer wall of, a 2L-volume container for dissolving the composition for digestive tract imaging. It would be more convenient if a dissolving liquid such as mineral water is previously filled in the container to be used for the dissolution.

Moreover, it is also possible to attach the above-mentioned blister pack to a container filled with an intestinal tract cleansing liquid which contains an electrolyte, a water-soluble polymer, a sweetening agent, and a liquid perfume, and whose osmotic pressure is from 200 to 440 mOsm/L.

As another example, a bag for a kit, which could be referred to as a triple bag that can accommodate the respective ingredients and the composition of the liquid preparation for oral administration of the present invention, in three zones partitioned by fused portions in a flexible plastic bag, can be enumerated as a good example. By pressing this bag for a kit with pressure by the hands or the like at the time of use, the middle fused portions are peeled off, by which the respective powder ingredients or the composition can be mixed with the dissolving liquid to thereby prepare the liquid preparation for oral administration. In addition, if an instruction and a note of caution are printed on the surface of the bag for a kit, it is possible to prepare them as a kit with only one bag (Japanese Design Application No.2000-2619).

For a patient with an inflammatory bowel disease, the contrast agent may irritate the inflammation of the mucosa of the intestinal tract, which may cause an adverse effect on an ulcer and such a diseased part. Thus, the kit or the packaged product can include a steroid drug for the purpose of preventing inflammation.

### [Examples]

Next is a more detailed description of the present invention with reference to Examples. However, the present invention is not to be limited by the following Examples.

### [Example 1]

Ten voluntary examinees were subjected to the pretreatment with use of the liquid preparation for oral administration of the present invention, and then to the CT colonography.
Specifically speaking, a colon cleansing solution mix prepared by adding a positive contrast agent comprising an iodine compound as an active ingredient to an oral intestinal tract cleansing liquid comprising polyethylene glycol as a main active ingredient (hereunder, may be referred to as a PEG solution) was used as the liquid preparation for oral administration of the present invention. Niflec (registered trademark) (a product of Ajinomoto Pharma) was used as the PEG solution. An oral/enema water-soluble iodine preparation (Gastrografin (registered trademark)) was used as the contrast agent.

### <Examinees>

Ten examinees had all experienced colon cleansing by taking 2L of PEG solution and colon endoscopy in the past. The gender of the examinees consisted of nine males and one female, and the age ranged from 36 years to 63 years old with the average age of 50.5 years. Regarding the body shape, the height ranged from 158.0 cm to 178.0 cm (average of 168.9cm), the weight ranged from 50.0 kg to 78.7kg (average of 67.5kg), and the body mass index (BMI) ranged from 20.0 to 27.1 (average of 23.6) (see Table 1). Regarding the usual defecation habit, five examinees were normal (excreted a formed stool once a day), five examinees had twice or more excretions a day or slight diarrhea (see Table 2).

**[Table 1]**

| | | |
|---|---|---|
| Gender | Male | Nine examinees |
| | Female | One examinee |
| Age | 36 years to 63 years old | |
| | Average 50.5 years old | |
| Height | 158 cm to 178 cm | |
| | Average | 168.9 cm |
| Weight | 50.0 kg to 78.7 kg | |
| | Average | 67.5 kg |
| BMI | 20.0 to 27.1 | |
| | Average | 23.6 |

**[Table 2]**

| Normal | Five examinees |
|---|---|
| Twice or more excretions a day or slight diarrhea | Five examinees |

### <Determination of iodine compound concentration in colon cleansing solution mix>

Gastrografin was added to Niflec to produce colon cleansing solution mixes having Gastrografin concentrations of 0.5%, 1%, 3%, and 5% (hereunder, may be simply referred to as "0.5% colon cleansing solution mix" and the like). The iodine contents in the above-mentioned colon cleansing solution mixes were respectively 1.85 mg/ mL in the 0.5% colon cleansing solution mix, 3.7 mg/ mL in the 1% colon cleansing solution mix, 11.1 mg/mL in the 3% colon cleansing solution mix, and 18.5 mg/ mL in the 5% colon cleansing solution mix. In order to stimulate a condition of the human body as close as possible, the CT image was taken in a state where plastic bottles containing these colon cleansing solution mixes were sandwiched by acrylic resins having a thickness of 5.0 cm from the top and the bottom. The CT device used herein was the Aquilion TSX-101A/4E (a product of Toshiba).

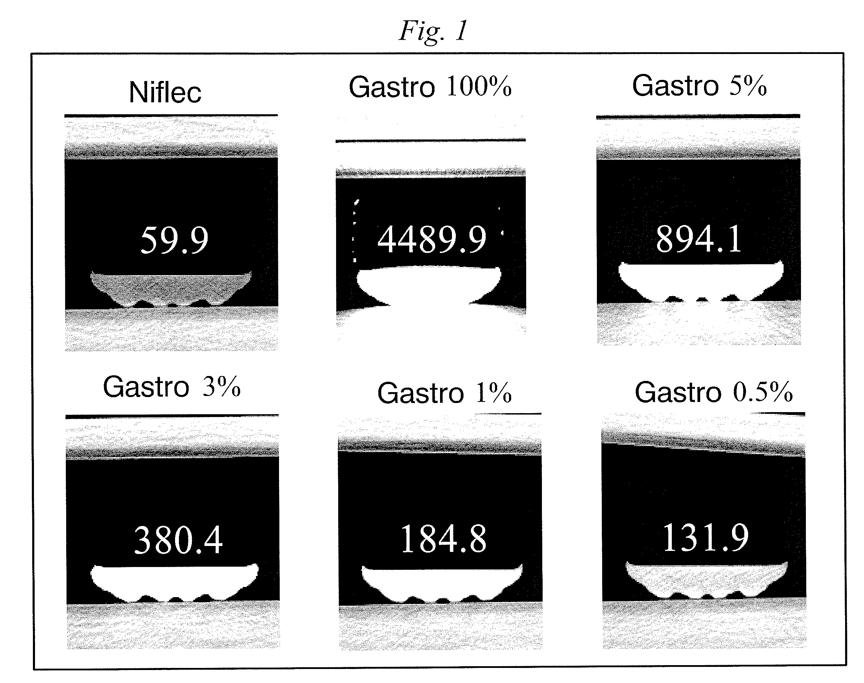

The results of the CT imaging are shown in FIG. 1. In FIG. 1, the term "Gastro 100%" denotes the result of the CT imaging of the plastic bottle injected with only the Gastrografin undiluted solution, and the term "Gastro X%" denotes the result of the CT imaging of the plastic bottle injected with the X% colon cleansing solution mix. As a result, the CT value of the 0.5% colon cleansing solution mix was 131.9, the CT value of the 1% colon cleansing solution mix was 184.8, the CT value of the 3% colon cleansing solution mix was 380.4, and the CT value of the 5% colon cleansing solution mix was 894.1. At this time, the CT value of water was 38.7, the CT value of Niflec was 59.9, and the CT value of the Gastrografin undiluted solution was 4489.9. 2D CT images produced under a condition where the voltage was 120 kV, the current was 100 mA (50 mAs), and the slice thickness was 3 mm were slightly blurred when using the 0.5% colon cleansing solution mix as compared to Niflec, although the contrast effect was clear. With the 1 % colon cleansing solution mix and the 3% colon cleansing solution mix, the contrast effect was clearly visible. On the other hand, the 5% colon cleansing solution mix was so dense that artifact was generated. Considering these results and the taste of Gastrografin, the concentration of Gastrografin in the colon cleansing solution mix for use in the following investigations was determined to be 1 %.

### <Determination of iodine compound concentration in colon cleansing solution mix>

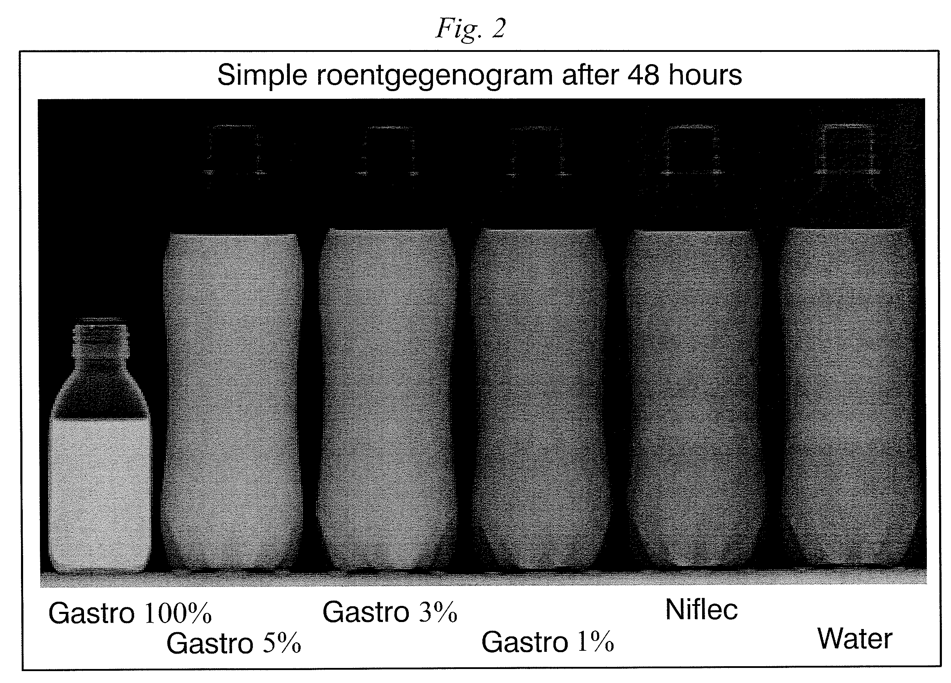

In order to investigate whether or not the contrast effect was deteriorated due to separation and precipitation of Niflec and Gastrografin in the colon cleansing solution mix with the passage of time, a simple roentgenogram of the 1% colon cleansing solution mix injected into a plastic bottle was taken just after the preparation and 48 hours later.
The results of the thus taken simple roentgenogram after 48 hours are shown in FIG. 2. In FIG. 2, the terms "Gastro 100%" and "Gastro X%" have the same meanings as those of FIG. 1. As a result, separation and precipitation of Gastrografin were not found in the 1% colon cleansing solution mix 48 hours after the preparation.

### <Pretreatment with use of 1% colon cleansing solution mix and CT imaging>

A total amount of 2000 mL of 1% colon cleansing solution mix was prepared by adding 20 mL of Gastrografin to 1980 mL of Niflec. This 1% colon cleansing solution mix was taken by the ten examinees, respectively. After the defecation had been completed and the drained water had become transparent, the CT image taken.
In the insufflation to inflate the intestinal tract, a carbon dioxide gas (CO₂) was supplied by using a hand operated double balloon pump through the transanal route. The gas for inflating the intestinal tract was preferably carbon dioxide gas because it can be absorbed from the mucosa of the intestinal tract much more quickly than air, as well as giving little feeling of fullness to the examinees. No anticonvulsive agent was used.

The procedure of the imaging was such that: firstly, the posture of the examinee was set to the left lateral decubitus position; a tube for insufflation was inserted; and a gas was supplied to confirm that smooth insufflation was possible. Next, the posture of the examinee was set to the abdominal position; the insufflation was conducted until the examinee had claimed a strong feeling of fullness or the pressure inside the intestinal tract had reached 25 mmHg; scanography was performed to confirm that the large intestine had been sufficiently inflated; and thereafter the CT image was taken. After taking the image of the abdominal position, the posture of the examinee was set to the dorsal position, and scanography was again performed to take the CT image in the dorsal position. At this time, if the inflation of the large intestine had been insufficient, additional insufflation was conducted. After the completion of the CT image taking, colon endoscopy was conducted to see the circumstances of the residual feces and residual liquid in the large intestine by eye. The CT image was taken under the charge of a clinical X-ray technician.

### <Acceptability of 1% colon cleansing solution mix>

All the examinees were able to drink the total amount of 2000 mL of the 1% colon cleansing solution mix. The circumstances of drinking and the characteristics of defecation were recorded by the examinees themselves. The time required from the beginning to the completion of drinking was 50 minutes at the shortest and 175 minutes at the longest, with the average of 129 minutes.
After the completion of drinking of 2000 mL of the 1% colon cleansing solution mix, an interview investigation was conducted regarding the taste and the easiness to drink so as to compare with Niflec which had been experienced in the past. The results of this investigation are shown in Table 3. At first, regarding the taste, the Gastrografin undiluted solution was so bitter that it was quite hard to drink; however, all the examinees answered that the 1% colon cleansing solution mix had a better taste than Niflec without Gastrografin. The reason can be considered to be such that the balance between the bad taste of the polyethylene glycol and sodium sulfate mixed in Niflec and the slightly bitter taste of the adequately diluted Gastrografin was so appropriate that the salty taste of Niflec and the bitter taste of Gastrografin compensated with each other. Regarding the easiness to drink as a total of the taste and the quantity, the easiness to drink was apparently improved. For example, an examinee who had once been able to drink as little as 1500 mL of Niflec when receiving an endoscopic examination in the past, succeeded to drink 2000 mL of the 1% colon cleansing solution mix.

**[Table 3]**

| | 5 | 4 | 3 | 2 | 1 | Total point | Average |
|---|---|---|---|---|---|---|---|
| Smell | Ignorable | | | | Noticeable | | |
| | 80% | 10% | 10% | 0% | 0% | 47 | 4.7 |
| Taste | Excellent | | | | Bad | | |
| | 20% | 20% | 50% | 10% | 0% | 35 | 3.5 |
| Easiness to drink | Easy to drink | | | | Hard to drink | | |
| | 30% | 50% | 10% | 10% | 0% | 40 | 4.0 |
| Remaining taste | Excellent | | | | Bad | | |
| | 20% | 20% | 40% | 20% | 0% | 34 | 3.4 |
| Overall rating | Excellent | | | | Bad | | |
| | 37.5% | 25.0% | 27.5% | 10.0% | 0.0% | 156 | 3.9 |

### <Colon cleansing effect of 1% colon cleansing solution mix>

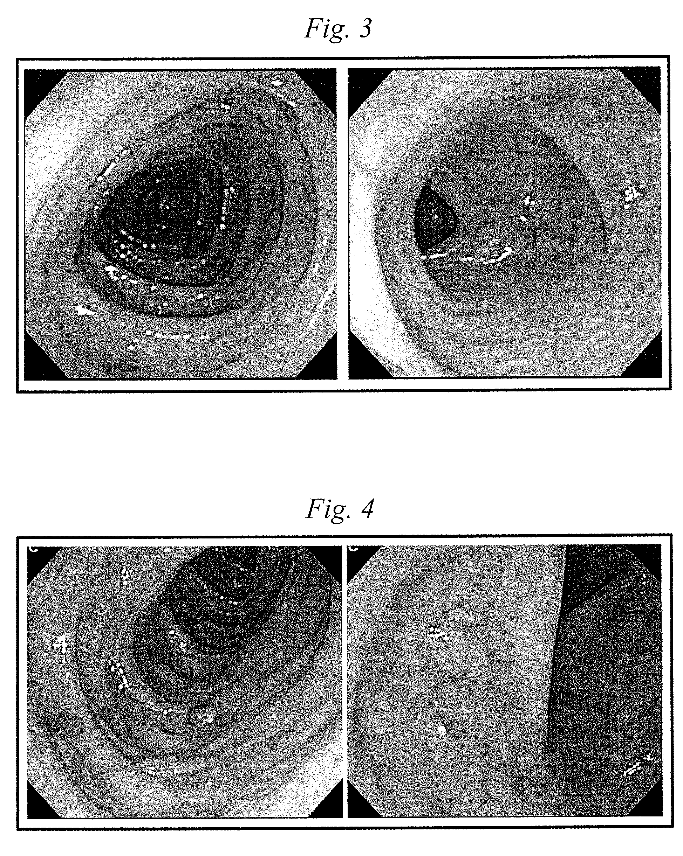

Regarding the circumstances of defecation and the characteristics of drained water, firstly, eight examinees (80.0%) had defecation prior to the beginning of drinking the 1 % colon cleansing solution mix.
The amount of the 1% colon cleansing solution mix at a time when the defecation was seen for the first time after the beginning of drinking was 500 mL for four examinees (44.4%), 1000 mL for four examinees (44.4%), and 1800 mL for one examinee (11.2%).
The amount of the 1% colon cleansing solution mix at a time when the drained water became transparent was 1000 mL for one examinee (11.1 %) and 1800 mL for eight examinees (88.9%).
Regarding the circumstances of residual feces at the time of the endoscopic examination after the completion of the CT image taking, no residual feces was found with only transparent drained water in seven examinees (70.0%), and little residual feces were found in three examinees (30.0%). Endoscopic images for an examinee with no residual feces and only transparent drained water are shown in FIG. 3 as an example. Endoscopic images for an examinee with little residual feces are shown in FIG. 4 as an example.

### <Acceptability of CO₂ insufflation>

All the examinees had a feeling of fullness and a stimulating feeling of anus (feeling of defecation) by insufflation. Five examinees (50.0%) felt abdominal pains. Although CT image taking required about 20 second of holding the breath, no examinee had a particularly suffering feeling. In addition, as a comparison with the endoscopic examination conducted by intravenous injection of 35 mg of pethidine hydrochloride and 10 mg of scopolamine butylbromide after the CT image taking, all the examinees answered that the endoscopic examination was easier.

### <Contrast effect of 1% colon cleansing solution mix>

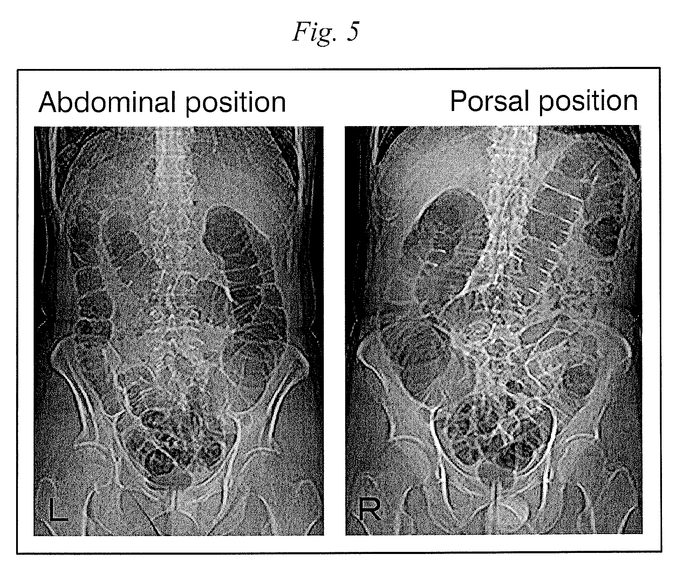

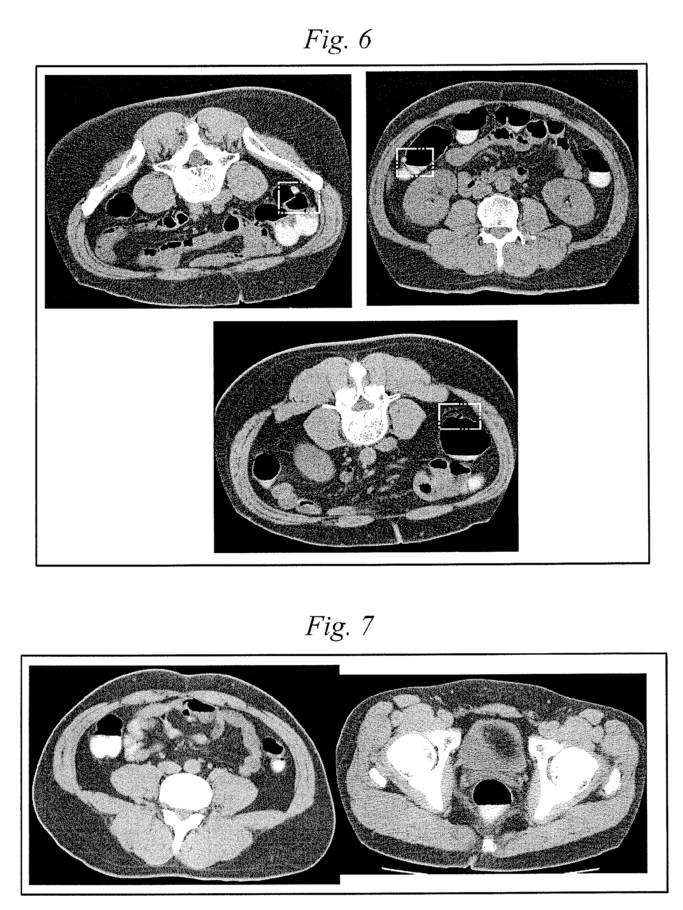

Firstly, CO₂ insufflation was conducted and scanographic images were examined just before the CT image taking. The scanographic images are shown in FIG. 5. In the abdominal position, the whole large intestine was sufficiently inflated with CO₂. In the dorsal position, the rectum, the sigmoid colon, the transverse colon, the ascending colon, and the cecum were sufficiently inflated with CO₂. Only the descending colon was insufficiently inflated.
2D images captured by CT image taking are shown in FIGs. 6 and 7. In the CT images, gas pattern images which can satisfy the 2D interpretation were obtained almost throughout the whole large intestine by combining the abdominal position and the dorsal position. In addition, the contrast of a pool of liquid content defining the air fluid level in the intestinal tract inflated with CO₂ was enhanced by Gastrografin, which made it possible to discriminate from the wall of the intestinal tract and the residual feces. In FIG. 6, circular low CT value regions show fecal pat effusing from the diverticulum. The CT value of the pooled liquid was 150 in the sigmoid colon, 160 in the descending colon, 160 in the transverse colon, 160 in the ascending colon, and 180 in the cecum. In the rectum, a phenomenon in which the CT value was lowered to 120, which made the visual observation difficult, was seen (FIG. 7). The reason can be suggested to be such that the 1% colon cleansing solution mix was diluted with a secretory fluid from the large intestine.

### <Quality of CT images>

Regarding the quality of images captured by four multidectector-row helical CT, firstly, the inflation of the intestinal tract was good so long as it was determined by scanography and 2D images, although any anticonvulsive agent was not used. The CT value of the pooled liquid was from 120 (rectum) to 180 (cecum), meaning that the CT value did not reached to 200 that can offer a favorable electronic cleansing effect. However, in terms of visual observation, the pooled liquid was able to be discriminated from the surrounding normal tissue and fecal pat. That is, it was suggested that the method of conducting the CT image taking after the pretreatment with use of the liquid preparation for oral administration of the present invention was a useful method for 2D image diagnosis, although it is not enough to enhance the contrast for the creation of VE or such 3D images for detecting a lesion.

The 2D image is an intact CT image without excessive modifications of computer software such as seen in VE images. By deliberately interpreting the tomograms of small slice pitches with full use of MPR and such techniques (2D primary), it is possible to conduct the large intestine cancer test with higher precision than the 3D primary which mainly uses VE for the interpretation. In addition, the 2D primary does not require the electronic cleansing which is a must for the 3D primary. This is because that the synergistic effect of CO₂ serving as a negative contrast agent and a positive contrast agent (iodine compound) enables to observe the whole periphery of the wall of the intestinal tract all at once in one slice of image.

### INDUSTRIAL APPLICABILITY

The liquid preparation for oral administration of the present invention can be suitably used for the pretreatment of CT colonography, and thus is applicable to the fields of clinical examinations such as medical checkups of the large intestine and the like. In particular, the liquid preparation for oral administration of the present invention is applicable to a first screening test to be conducted after a fecal occult blood test, on an examinee who has been found to be positive in the fecal occult blood test. Therefore, the present invention is extremely useful for the industry.

## Claims

1. A liquid preparation for oral administration in digestive tract imaging, wherein:
the preparation comprises
an iodine compound,
an electrolyte, and
a water-soluble polymer; and
the iodine content is 1.5 to 12.0 mg/ mL.

2. A liquid preparation for oral administration according to claim 1, wherein the preparation further comprises one or more types of ingredients selected from the group consisting of a sweetening agent and a perfume.

3. A liquid preparation for oral administration according to claim 1, wherein said water-soluble polymer is one or more types of water-soluble polymers selected from the group consisting of polyethylene glycol, polydextrose, dextran, dextrin, hydroxyethyl starch, gum arabic, pullulan, pectin, albumin, and carboxymethyl cellulose.

4. A liquid preparation for oral administration according to claim 1, wherein the mixed amount of said electrolyte is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L.

5. A liquid preparation for oral administration according to claim 1, wherein said perfume is a citrus-based perfume.

6. A liquid preparation for oral administration according to claim 1, wherein said sweetening agent comprises one or more types of saccharides selected from the group consisting of sucrose, sorbitol, xylitol, erythritol, mannitol, trehalose, lactitol, lactulose, maltitol, palatinose, raffinose, and glycerin.

7. A liquid preparation for oral administration according to claim 1, wherein: the preparation is to be taken from the beginning of intestinal tract cleansing as a pretreatment of CT colonography; a CT image is taken after the completion of the intestinal tract cleansing; and digestive tract tissue of the large intestine is visualized from the thus taken CT 2D image.

8. A composition for digestive tract imaging for use in intestinal tract cleansing, wherein: the composition comprises
an iodine compound,
an electrolyte, and
a water-soluble polymer; and
the iodine content is 1.5 to 12.0 mg/ mL when dissolved in water.

9. A composition for digestive tract imaging according to claim 8, wherein:
the content of said electrolyte when dissolved in water is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L; and
the composition comprises one or more types of water-soluble polymers selected from the group consisting of polyethylene glycol, polydextrose, dextran, dextrin, hydroxyethyl starch, gum arabic, pullulan, pectin, albumin, and carboxymethyl cellulose, as said water-soluble polymer; or,
one or more types of saccharides selected from the group consisting of sucrose, sorbitol, xylitol, erythritol, mannitol, trehalose, lactitol, lactulose, maltitol, palatinose, raffinose, and glycerin, as saccharide.

10. A composition for digestive tract imaging according to claim 8, wherein;
the content of said electrolyte when dissolved in water is such that Na⁺ accounts for 30 to 150 mEq/L, K⁺ accounts for 2 to 20 mEq/L, Cl⁻ accounts for 20 to 70 mEq/L, and HCO₃⁻ accounts for 10 to 50 mEq/L; and
the composition comprises one or more types of water-soluble polymers selected from the group consisting of polyethylene glycol, polydextrose, dextran, dextrin, hydroxyethyl starch, gum arabic, pullulan, pectin, albumin, and carboxymethyl cellulose, as said water-soluble polymer; and
one or more types of saccharides selected from the group consisting of sucrose, sorbitol, xylitol, erythritol, mannitol, trehalose, lactitol, lactulose, maltitol, palatinose, raffinose, and glycerin, as saccharide.

11. A composition for digestive tract imaging according to claim 8, wherein the composition further comprises one or more types of ingredients selected from the group consisting of a sweetening agent and a perfume.

12. A composition for digestive tract imaging according to claim 8, wherein:
said intestinal tract cleansing is a pretreatment of CT colonography; and 0.5 to 4L of said water solution is orally administered from the beginning of the intestinal tract cleansing;
a CT image is taken after the completion of said intestinal tract cleansing; and
digestive tract tissue of the large intestine is visualized from the thus taken CT 2D image.

13. A pretreatment agent for CT colonography examination, wherein: the agent comprises a combination of an oral intestinal tract cleansing agent and an iodine contrast agent; and the iodine content is adjusted to 1.5 to 12.0 mg/ mL and thereafter the intestinal tract cleansing is begun by starting oral administration.

14. A use of a liquid preparation for oral administration in digestive tract imaging, wherein: the preparation comprises
an iodine compound,
an electrolyte, and
a water-soluble polymer; and
the iodine content is 1.5 to 12.0 mg/ mL.

15. A use of a liquid preparation for oral administration in intestinal tract cleansing, wherein: the preparation comprises
an iodine compound,
an electrolyte, and
a water-soluble polymer; and
the iodine content is 1.5 to 12.0 mg/ mL.

16. An oral use of a combination of an oral intestinal tract cleansing agent and an iodine contrast agent in a pretreatment for CT colonography examination, wherein: the use of said combination is for intestinal tract cleansing and digestive tract imaging; and the content of the iodine compound is 1.5 to 12.0 mg/ mL in said combination of the oral intestinal tract cleansing agent and the iodine contrast agent.

17. A kit for digestive tract imaging or intestinal tract cleansing, wherein: the kit comprises
an iodine compound,
an electrolyte, and
a water-soluble polymer; and
the iodine content is 1.5 to 12.0 mg/ mL.
